# EUROPEAN PATENT APPLICATION

(11) **EP 2 301 420 A1**
(43) Date of publication of application: **30.03.2011**
(21) Application number: 09766476.7
(22) Date of filing: 13.03.2009
(51) Int. Cl.: A61B 1/06, F21S 2/00, G02B 23/26, F21Y 101/02

(54) **LIGHT SOURCE DEVICE AND ENDOSCOPIC DEVICE**

(30) Priority: 17.06.2008 JP 2008158148
(71) Applicant: Olympus Corporation, Shibuya-ku Tokyo 151-0072 (JP)
(72) Inventor: GODO, Hirokazu, Tokyo 151-0072 (JP)
(74) Representative: Schicker, Silvia
(86) International application number: PCT/JP2009/054841
(87) International publication number: WO 2009/154028

(57) **Abstract**

An object is to provide a light source device that can readily synchronize the lighting cycle of light sources and the rotation cycle of a light guide rod. Provided is a light source device (10) that includes LEDs (11) arranged in the form of a ring and each configured to emit illumination light in a radially inward direction of the ring; a light guide member (15) that optically guides the illumination light, emitted from each LED (11), along a central axis of the ring; a rotary motor that rotationally drives the light guide member (15) about the central axis; a reflective photo-sensor that detects rotation of the rotary motor; a CCD (5) that detects an amount of the illumination light optically guided by the light guide member (15); and a control section (6) that controls these sections and adjusts a phase difference between a lighting signal, which is used for performing lighting control of the LEDs (11), and a rotation detection signal, which is detected by the reflective photo-sensor, on the basis of the amount of light detected by the CCD (5).

## Description

### Technical Field

The present invention relates to a light source device equipped with a plurality of light sources, and to an endoscopic apparatus.

### Background Art

In the related art, there is a known light source device that sequentially turns on light sources, such as LEDs, arranged in the form of a ring with high output and rotates a light guide rod in accordance with the lighting cycle of the light sources so as to emit high-intensity illumination light (for example, see Patent Citation 1). This light source device is equipped with detecting means for detecting the illumination light from the LEDs and uses this detecting means to perform lighting control of the LEDs.
Patent Citation 1:
   The Publication of Japanese Patent No. 3989302

### Disclosure of Invention

However, in the technology disclosed in Patent Citation 1 described above, a specific method for synchronizing the lighting cycle of the LEDs and the rotation cycle of the light guide rod is not disclosed.
For example, if the lighting cycle of the LEDs is to be synchronized with one frame of a video signal, it is necessary to additionally adjust the phase difference between the video signal and the lighting timing of the LEDs. There is also a problem in that, should the user need to perform adjustment when the lighting cycle of the LEDs and the rotation cycle of the light guide rod deviate from each other due to degradation over time or the like, this adjustment is difficult.

In view of the circumstances described above, an object of the present invention is to provide a light source device that can readily synchronize the lighting cycle of light sources and the rotation cycle of a light guide rod.

In order to achieve the aforementioned object, the present invention employs the following solutions.
A first aspect of the present invention provides a light source device that includes a plurality of light sources arranged in the form of a ring and each configured to emit illumination light in a radially inward direction of the ring; a light guide section that optically guides the illumination light, emitted from each light source, along a central axis of the ring; a rotating section that rotationally drives the light guide section about the central axis; a rotation detecting section that detects rotation of the rotating section; a light detecting section that detects an amount of the illumination light optically guided by the light guide section; and a control section that controls the sections and adjusts a phase difference between a lighting signal, which is used for performing lighting control of the light sources, and a rotation detection signal, which is detected by the rotation detecting section, on the basis of the amount of light detected by the light detecting section.
In this case, the rotation detection signal generated by the rotation detecting section by detecting the rotation of the rotating section may either be a signal that indicates a rotational angle or an index signal that indicates that one rotation has been made. If a signal that indicates a rotational angle is used, the rotation detecting section requires an expensive detector such as a rotary encoder, whereas if an index signal is used, since the rotation detecting section simply needs to be capable of detecting that one rotation has been made, a simpler detector can be used.

With the first aspect of the present invention, the amount of illumination light emitted from the plurality of light sources and optically guided by the light guide section is detected by the light detecting section, and the phase difference between the lighting signal used for performing lighting control of the light sources and the rotation detection signal detected by the rotation detecting section is adjusted by the control section on the basis of the detected amount of light.
A specific method for adjusting the phase difference may be a method of adjusting the phases of both the lighting signal and the rotation detection signal relative to a certain reference signal, or a method of adjusting the phase of one of the signals while fixing the phase of the other signal as a reference signal. For example, by adjusting the phase difference after the rotating section runs at a constant speed, the phase of the rotation detection signal can be fixed relative to the reference signal so that the phase difference between the lighting signal and the rotation detection signal can be readily adjusted by simply adjusting the phase of the lighting signal alone.
Consequently, the lighting cycle of the light sources and the rotation cycle of the rotating section can be synchronized with each other, thereby allowing for emission of a stable amount of illumination light.

In the aforementioned aspect, the control section may adjust the phase difference by making each light source emit the illumination light for a length of time that is equal to a length of time for which the illumination light from the light source enters the light guide section when the light source is constantly turned on.
By making each light source emit the illumination light for a length of time that is equal to a length of time for which the illumination light from the light source enters the light guide section when the light source is constantly turned on, the deviation between the phase of the lighting signal and the phase of the rotation detection signal can be responsively reflected in the amount of illumination light optically guided by the light guide section. Therefore, by detecting this amount of illumination light using the light detecting section, the phase difference between the lighting signal and the rotation detection signal can be adjusted with high accuracy.

In the aforementioned aspect, the control section may adjust the phase difference by setting the amount of light to be lower than the amount of light during illumination.
For example, the amount of light from the light sources, such as LEDs, decreases with increasing temperature. Therefore, when the light sources are driven at a maximum output, the amount of light is significantly reduced due to heat. If this phenomenon occurs when the phase difference between the lighting signal and the rotation detection signal is being adjusted, it becomes impossible to determine whether a change in the amount of illumination light detected by the light detecting section is caused by the phase difference or heat, making it difficult to adjust the phase difference properly.
Therefore, by setting the amount of light during the phase-difference adjustment to be lower than the amount of light during illumination, the heat generated by the light sources can be reduced so that a reduction in the amount of light caused by heat can be suppressed, whereby the phase difference between the lighting signal and the rotation detection signal can be adjusted with high accuracy.

In the aforementioned aspect, the control section may adjust the phase difference by turning on one of or some of the light sources.
This allows for less heat to be generated by the light sources so that a reduction in the amount of light caused by heat can be suppressed, whereby the phase difference between the lighting signal and the rotation detection signal can be adjusted with high accuracy.

In the aforementioned aspect, the control section may adjust the phase of the lighting signal by varying the phase of the lighting signal in a state where the light guide section is rotated at a constant speed by the rotating section, so that the amount of light detected by the light detecting section is at a maximum.
Accordingly, the phase difference between the lighting signal and the rotation detection signal can be readily adjusted.

In the aforementioned aspect, the light source device may further include a storage section that stores the phase difference adjusted by the control section, and the control section may control the sections on the basis of the phase difference stored in the storage section when power is turned on or reset.
Accordingly, the light source device can be operated on the basis of the pre-adjusted phase difference between the lighting signal and the rotation detection signal.

A second aspect of the present invention provides an endoscopic apparatus equipped with the aforementioned light source device.
With the second aspect of the present invention, the phase difference between the lighting signal and the rotation detection signal can be adjusted on the basis of light optically received by an image acquisition device of the endoscopic apparatus. The image acquisition device, which is provided at a tip of a scope of the endoscopic apparatus, may also be used as a light detecting section.

The present invention is advantageous in that the lighting cycle of light sources and the rotation cycle of a light guide rod can be readily synchronized with each other.

### Brief Description of Drawings

[FIG. 1] Fig. 1 is a schematic diagram of a light source device according to an embodiment of the present invention.
[FIG. 2] Fig. 2 is a flow chart illustrating an operation performed when a phase-difference adjustment mode is executed.
[FIG. 3] Fig. 3 illustrates a drive sequence of LEDs in the phase-difference adjustment mode.
[FIG. 4A] Fig. 4A is a flow chart illustrating processing performed in the phase-difference adjustment mode.
[FIG. 4B] Fig. 4B is a flow chart illustrating processing performed in the phase-difference adjustment mode.
[FIG. 5] Fig. 5 is a schematic configuration diagram of an endoscopic apparatus to which the light source device in Fig. 1 is applied.
[FIG. 6] Fig. 6 is a partly enlarged view of the endoscopic apparatus in Fig. 5.
[FIG. 7] Fig. 7 is a partly enlarged view of the endoscopic apparatus in Fig. 5.

### Explanation of Reference:

- 1:: endoscopic apparatus
- 5:: CCD
- 6:: control section
- 7:: storage section
- 10:: light source device
- 11:: LED
- 15:: light guide member
- 20:: scope
- 22:: CCD
- 23:: image processor
- 24:: monitor
- 40:: cap

### Best Mode for Carrying Out the Invention

An illumination device according to an embodiment of the present invention will be described below with reference to the drawings.

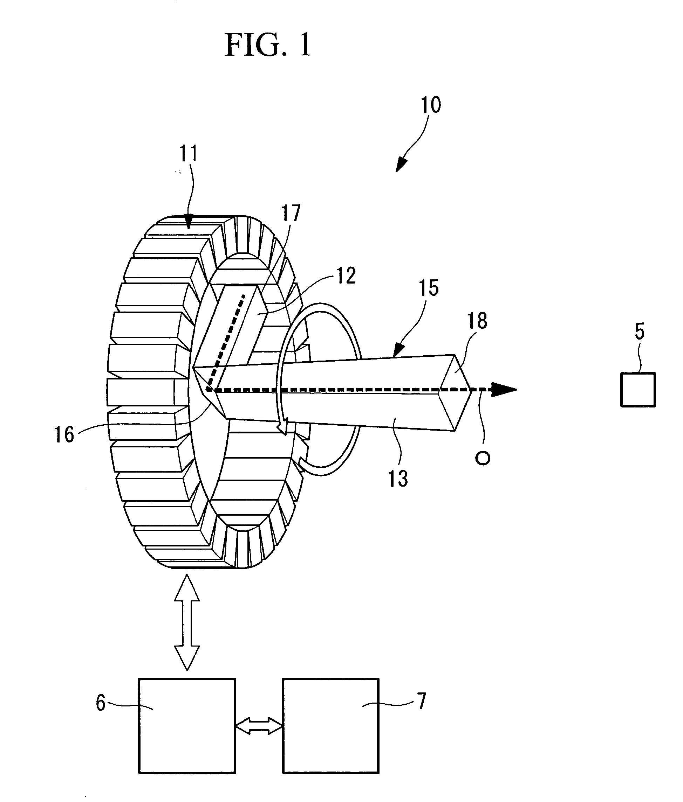

Fig. 1 is a schematic diagram explaining the configuration of the illumination device according to this embodiment.
As shown in Fig. 1, a light source device 10 according to this embodiment includes a plurality of LEDs (light sources) 11 arranged in the form of a ring, a light guide member (light guide section) 15 disposed radially inward of the ring, a rotary motor (rotating section) (not shown) that rotationally drives the light guide member 15 about a central axis of the ring, a rotation sensor (rotation detecting section) (not shown) that detects the rotation of the rotating section, a CCD (light detecting section) 5 disposed on a light emission axis of the light guide member 15, a control section 6 that controls these sections, and a storage section 7 that is bidirectionally connected to the control section 6.

The LEDs 11 are arranged in the form of a ring and are each configured to emit illumination light in a radially inward direction of the ring.
The light guide member 15 includes a light guide rod 12 that optically guides the illumination light, emitted from each LED 11, in a radially inward direction of the ring; a prism 16 that reflects the illumination light, optically guided by the light guide rod 12, to a direction extending along a central axis O of the ring; and a light guide rod 13 that optically guides the illumination light reflected by the prism 16. With such a configuration, the light guide member 15 is configured to optically guide the illumination light, emitted from each LED 11, along the central axis O of the ring.

The rotary motor is disposed along the central axis O of the light guide member 15 and is configured to rotationally drive the light guide member 15 about the central axis O.
The CCD 5 is a photoelectric converter that converts light energy into electric power and is configured to detect the amount of illumination light optically guided by the light guide member 15. Moreover, the CCD 5 sends, to the control section 6, a reference signal used as a reference for phase adjustment between the rotation of the rotary motor and the lighting cycle of the LEDs.

The storage section 7 is, for example, an EEPROM capable of keeping data even when the power is turned off, and is configured to store information such as a phase difference adjusted by the control section 6.
The rotation sensor is constituted of a reflective photo-sensor (photo-reflector) (not shown) and generates an index signal (rotation detection signal) by detecting one rotation of the rotating section as one cycle.

In addition to controlling the above-described sections, the control section 6 is configured to adjust the phase difference between a lighting signal, which is used for performing lighting control of the LEDs 11, and the rotation detection signal, detected by the rotation sensor, relative to the reference signal on the basis of the amount of light detected by the CCD 5.

In this case, although the phases of both the lighting signal and the rotation detection signal are adjusted relative to the reference signal generated by the CCD 5 as a specific method for adjusting the phase difference, the phase of one of the lighting signal and the rotation detection signal may be adjusted by using the phase of the other signal as a reference signal. For example, by adjusting the phase difference after the rotary motor runs stably at a constant speed, the phase of the rotation detection signal can be fixed relative to the reference signal so that the phase difference between the lighting signal and the rotation detection signal can be readily adjusted by simply adjusting the phase of the lighting signal alone.

Furthermore, the control section 6 is configured to read the information, such as the phase difference, stored in the storage section 7 when the power is turned on or reset so as to control the individual sections on the basis of the read information.
Accordingly, the light source device 10 can be activated on the basis of the pre-adjusted phase difference between the lighting signal and the rotation detection signal.

Normal operation of the light source device 10 having the above-described configuration will be described below.
When a command for driving the light source device 10 is given, the control section 6 outputs a control signal for rotationally driving the rotary motor, and the rotation sensor detects the rotation of the light guide member 15.

The control section 6 performs lighting control of the LEDs 11 on the basis of the rotation detection signal detected by the rotation sensor. Specifically, based on an LED lighting signal that is given a phase difference set in accordance with a phase-difference adjustment mode, to be described later, relative to the reference signal, the control section 6 supplies electric power to the LED that faces an entrance face 17 of the light guide member 15 so as to make the LED emit the illumination light. In this case, the LEDs 11 are controlled such that each LED is pulse-lit by being driven instantaneously with an electric current that is higher than that in a rated driving mode so as to emit high-intensity illumination light.

Consequently, the illumination light is emitted from each LED 11, and the illumination light entering through the entrance face 17 is optically guided within the light guide member 15 so as to be emitted from an exit face 18 along a light emission axis O. The amount of illumination light emitted in this manner is detected by the CCD 5. In this case, the light from the LEDs appears to be output in a superimposed manner.

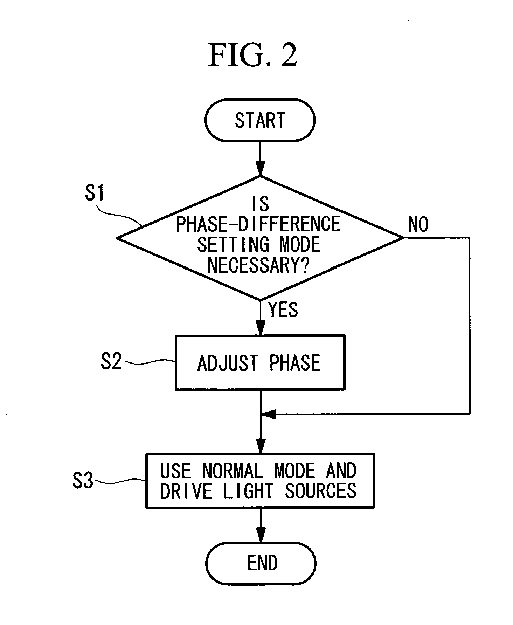

Next, an operation performed when the light source device 10 executes a phase-difference adjustment mode will be described below using a flow chart shown in Fig. 2.
First, it is determined whether or not a phase-difference setting mode is necessary on the basis of a predetermined condition (S1). Examples of such a predetermined condition include when the power of the light source device 10 is turned on and when the cumulative operating time of the light source device 10 reaches a predetermined value.

If it is determined that the phase-difference setting mode is necessary, the phase-difference setting mode is commenced so as to adjust the phase difference between the lighting signal and the rotation detection signal relative to the reference signal (S2). A specific method for adjusting the phase difference will be described later.
If it is determined that the phase-difference setting mode is not necessary or when the phase-difference adjustment is completed, the phase-difference setting mode is terminated so as to switch to a normal illumination mode (S3). This switching between modes may be performed automatically or manually.

Next, the specific method for adjusting the phase difference between the lighting signal and the rotation detection signal will be described below.

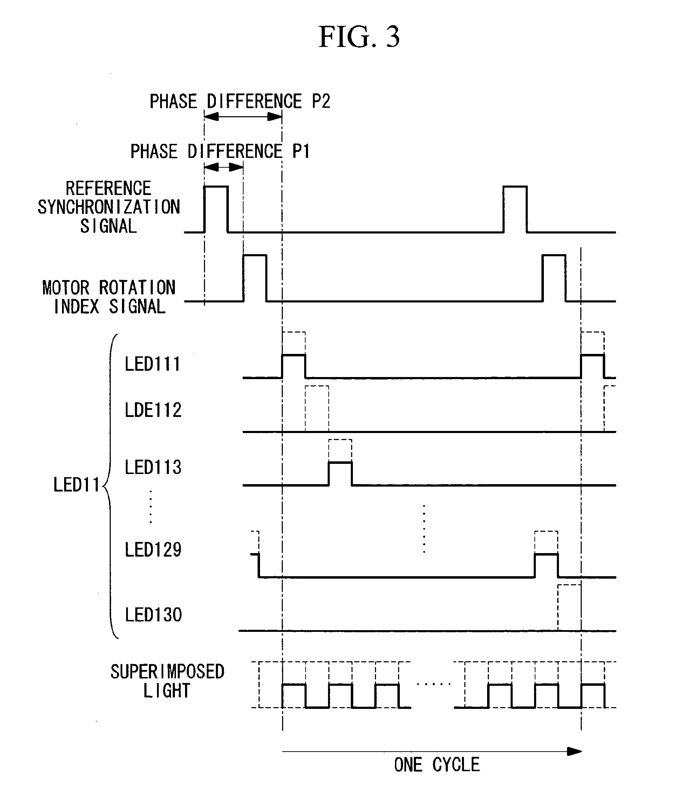

Fig. 3 illustrates a drive sequence of the LEDs 11 in the phase-difference adjustment mode. Here, LEDs 111 to 130 arranged in the form of a ring are controlled by the control section 6 so as to sequentially emit light in pulses.

In Fig. 3, control is performed so that a phase difference between a reference synchronization signal (reference signal) and a motor rotation index signal (rotation detection signal) is equal to a predetermined value P1. Furthermore, a phase difference between the reference synchronization signal and a reference-LED lighting signal is equal to P2. The following description is directed to a case where the LED 111 is selected as a reference LED. The amount of superimposed light shown in Fig. 3 is the amount of illumination light optically guided by the light guide member 15 from each LED.

Regarding each LED, an output shown with a dashed line is the maximum output under the normal illumination mode. In contrast, the maximum output under the phase-difference setting mode is shown with a solid line. In the phase-difference setting mode, a period assigned to each LED for light emission, that is, a length of time for which the illumination light is emitted from each LED, is set equal to a length of time for which the illumination light from the LED enters the light guide member 15 when the LED is constantly turned on.
Consequently, deviation between the phase of the lighting signal and the phase of the rotation detection signal can be responsively reflected in the amount of illumination light optically guided by the light guide member 15. By detecting the amount of illumination light using the CCD 5, the phase difference between the lighting signal and the rotation detection signal can be adjusted with high accuracy.

Furthermore, in the phase-difference setting mode, the amount of electric current supplied to each LED is reduced so that the amount of light from each LED is reduced relative to that in the normal illumination mode. Moreover, instead of turning on all of the LEDs, the number of LEDs to be turned on is controlled by, for example, turning on some of the LEDs, as shown in Fig. 3.
This allows for less heat to be generated by the LEDs so that a reduction in the amount of light caused by heat can be suppressed, whereby the phase difference between the lighting signal and the rotation detection signal can be adjusted with high accuracy.

Under the above-described conditions, the control section 6 adjusts the phase difference between the lighting signal and the rotation detection signal by adjusting the phase difference between the reference synchronization signal and the lighting signal, used for performing lighting control of the LEDs 11, on the basis of the amount of superimposed light in each cycle, that is, the accumulated amount of light detected by the CCD 5 while the light guide member 15 makes one rotation.

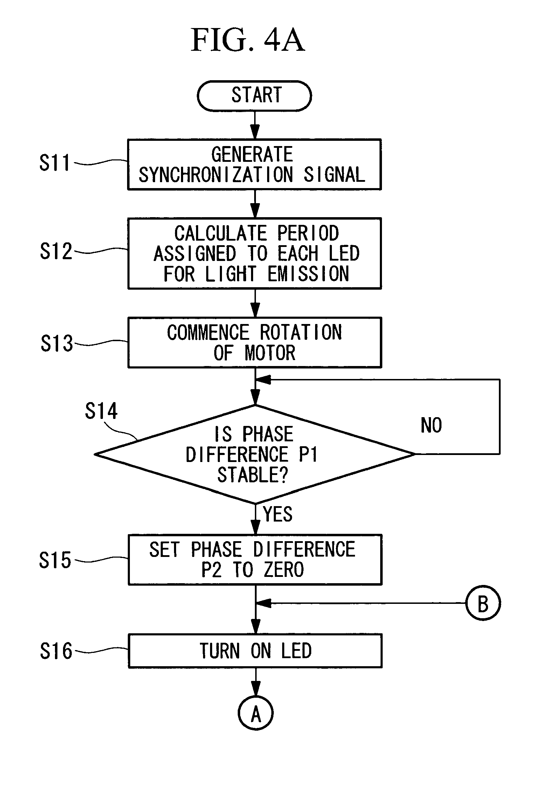

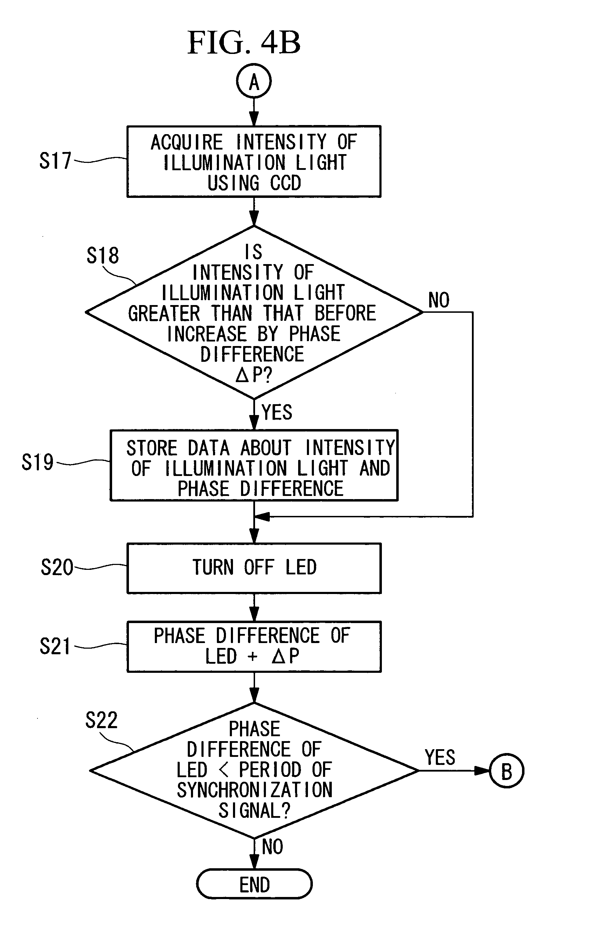

Next, specific processing performed in the phase-difference setting mode will be described below using a flow chart shown in Figs. 4A and 4B.
First, the light source device 10 is activated, and the synchronization signal acting as the reference signal is generated (S11). Then, a period assigned to each LED for light emission is calculated (S12), and rotational driving of the rotary motor is commenced (S13).
Subsequently, it is determined whether the phase difference P1 between the synchronization signal and the motor rotation index signal is stable, that is, the rotation of the rotary motor is stable (S14).

Next, in the state where the phase difference P1 is stable, the CCD 5 acquires the amount of illumination light when the phase difference P2 between the synchronization signal and the reference-LED lighting signal is incremented by ΔP.
First, the phase difference P2 is set to zero (S15), and the reference LED is turned on (S16). Then, the amount of illumination light at that time is acquired by the CCD 5 (S17), and it is determined whether or not the amount of illumination light after the phase difference P2 is increased by ΔP is greater than the amount of illumination light before the phase difference P2 is increased by ΔP (S18). If the amount of illumination light is increased due to the increase in the phase difference P2, the amount of illumination light at that time and the phase difference P2 are stored in the storage section 7 (S19), and the reference LED is turned off (S20).

Then, the phase difference P2 is increased by ΔP (S21), and the processing from S16 to S21 is repeated until the phase difference P2 becomes greater than or equal to the period of the synchronization signal (S22).
By performing the above-described processing, a phase difference P2 corresponding to a maximum amount of illumination light can be extracted.

As described above, with the light source device according to this embodiment, the amount of illumination light emitted from the plurality of LEDs and optically guided by the light guide member 15 is detected by the CCD 5, and the phase difference between the lighting signal and the rotation detection signal is adjusted by the control section 6 on the basis of the detected amount of light.
Thus, the lighting cycle of the LEDs and the rotation cycle of the rotary motor can be synchronized with each other, thereby allowing for emission of a stable amount of illumination light. Furthermore, by adjusting the phase difference between the lighting signal and the rotation detection signal so that the amount of illumination light detected by the CCD 5 is at a maximum, high-intensity illumination light can be emitted.
Furthermore, by fixing the phase of the rotary motor relative to the reference signal while varying the phase of the LED lighting signal in a state where the light guide member 15 is rotated at a constant speed by the rotary motor, the phase difference between the lighting signal and the rotation detection signal can be readily adjusted.

### [Example Application]

An example in which the above-described light source device 10 is applied to an endoscopic apparatus will be described below.

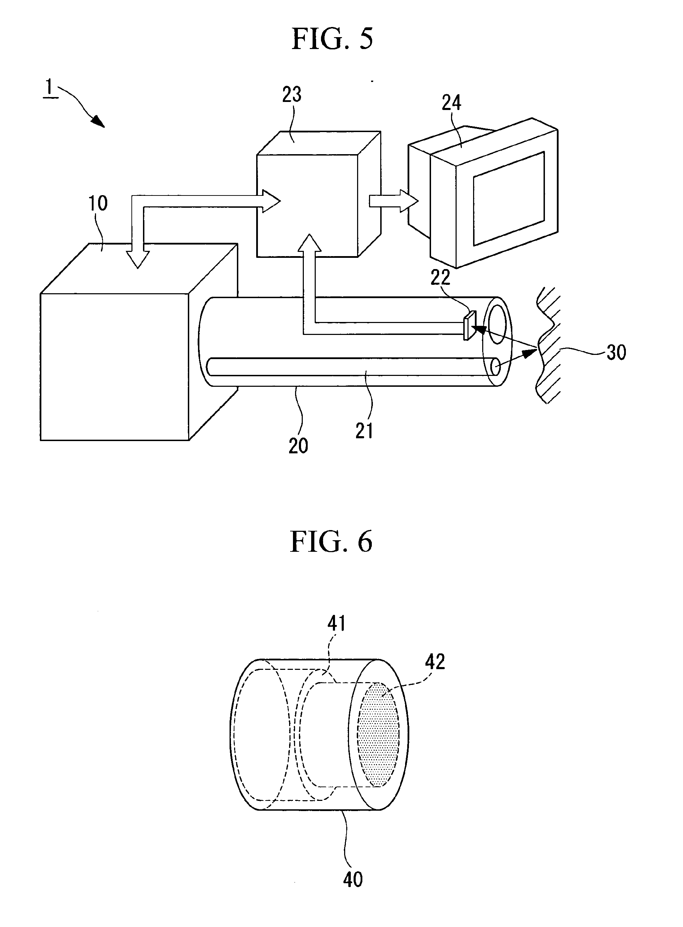

Fig. 5 is a schematic configuration diagram of an endoscopic apparatus 1 according to this example.
As shown in Fig. 5, the endoscopic apparatus 1 includes a light source device 10, a scope 20 coupled to the light source device, an image processor 23 that performs image processing on a video signal from the scope 20 so as to generate a display image, and a monitor 24 that displays the image.

The scope 20 contains a fiber 21 that optically guides illumination light from the light source device 10 to a tip of the scope 20 so as to illuminate an observation area 30 facing the tip of the scope 20 with the illumination light.
Furthermore, the tip of the scope 20 is provided with a CCD 22 that acquires an image of the observation area 30, and the image of the observation area 30 is output as a video signal to the image processor 23.

In the endoscopic apparatus 1 having the above-described configuration, an image-acquisition synchronization signal of the CCD 22 is used as a synchronization signal of the light source device 10. As mentioned above, the phase difference is adjusted in the light source device 10, and data about the phase difference is stored in the storage section 7, which does not erase the data even when the power is turned off. When the power is turned on or reset, the control section 6 reads the stored phase-difference data and uses it as a phase difference in the normal illumination mode.

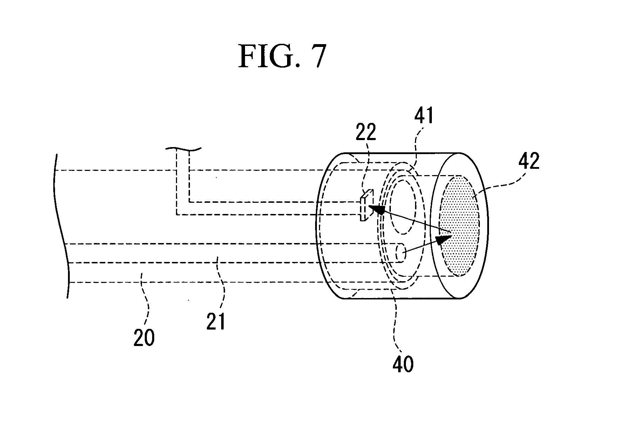

Fig. 6 illustrates a cap 40 that is to be attached at the time of the phase-difference setting mode.
The cap 40 includes an engagement section 41 that is to be fixed so as to cover the tip of the scope 20 and a target image-acquisition area 31 that is to serve as a photographic subject during phase adjustment. Attaching the cap 40 to the scope 20, as shown in Fig. 7, can eliminate the effect of external light and prevent fluctuations in the amount of light caused by fluctuations in the distance between the CCD 22 and the photographic subject during the phase-difference setting mode.

With the endoscopic apparatus 1 according to this example, the phase difference between the lighting signal and the rotation detection signal can be adjusted on the basis of the light optically received by the CCD 22 provided in the scope 20, thereby allowing for emission of a stable amount of illumination light.

Although the embodiment of the present invention has been described in detail above with reference to the drawings, specific configurations are not limited to this embodiment and include design alterations and the like so long as they do not depart from the spirit of the invention.
For example, in the above-described example, the synchronization signal of the light source device 10 may alternatively be a synchronization signal of a video signal displaying the light source device 10 as illumination light, or an index signal or a Hall-sensor signal obtained by detecting the rotation of the rotary motor. Furthermore, it is needless to say that the phase difference between the video signal and the rotation detection signal or the phase difference between the video signal and the LED lighting signal can sometimes be zero.
Furthermore, although a reflective photo-sensor (photo-reflector) is used as the rotation detecting section, a transmissive photo-sensor (photo-interrupter), a Hall sensor, a rotary encoder, or the like may be used as an alternative.
Furthermore, as a method of controlling the phase difference, the phase difference may be determined on the basis of the amount of light of multiple illumination light beams to take account of noise, or the peak amount of illumination light may be calculated by differentiating the amount of illumination light with respect to a phase-difference variation.

## Claims

1. A light source device comprising:
a plurality of light sources arranged in the form of a ring and each configured to emit illumination light in a radially inward direction of the ring;
a light guide section that optically guides the illumination light, emitted from each light source, along a central axis of the ring;
a rotating section that rotationally drives the light guide section about the central axis;
a rotation detecting section that detects rotation of the rotating section;
a light detecting section that detects an amount of the illumination light optically guided by the light guide section; and
a control section that controls the sections and adjusts a phase difference between a lighting signal, which is used for performing lighting control of the light sources, and a rotation detection signal, which is detected by the rotation detecting section, on the basis of the amount of light detected by the light detecting section.

2. The light source device according to Claim 1, wherein the control section adjusts the phase difference by making each light source emit the illumination light for a length of time that is equal to a length of time for which the illumination light from the light source enters the light guide section when the light source is constantly turned on.

3. The light source device according to Claim 1 or 2, wherein the control section adjusts the phase difference by setting the amount of light to be lower than the amount of light during illumination.

4. The light source device according to any one of Claims 1 to 3, wherein the control section adjusts the phase difference by turning on one of or some of the light sources.

5. The light source device according to any one of Claims 1 to 4, wherein the control section adjusts the phase of the lighting signal by varying the phase of the lighting signal in a state where the light guide section is rotated at a constant speed by the rotating section, so that the amount of light detected by the light detecting section is at a maximum.

6. The light source device according to any one of Claims 1 to 5, further comprising:
a storage section that stores the phase difference adjusted by the control section,
wherein the control section controls the sections on the basis of the phase difference stored in the storage section when power is turned on or reset.

7. An endoscopic apparatus comprising the light source device according to any one of Claims 1 to 6.
